# EUROPEAN PATENT APPLICATION

(11) **EP 1 990 349 A1**
(43) Date of publication of application: **12.11.2008**
(21) Application number: 07290582.1
(22) Date of filing: 09.05.2007
(51) Int. Cl.: C07K 14/56

(54) **Method for the production of high-level soluble human recombinant interferon alpha in E. coli and vectors useful for such a production**

(71) Applicant: INSTITUT PASTEUR, 75015 Paris (FR); INSTITUT PASTEUR DE TUNIS, 1002 Tunis (TN)
(72) Inventor: Fathallah, Mohamed Dahmani, Tunis Carthage 2035 (TN); Rabhi-Essafi, Imen, Megrine Coteauc 2033 (TN)
(74) Representative: Vialle-Presles, Marie José

(57) **Abstract**

Said method comprises the steps of:
(1) Transforming an *E. coli* selected in the group consisting of E. *coli* protease deficient host strains, and *E. coli* reductase deficient host strains, with a recombinant expression vector comprising the sequence encoding the glutathione-S-transferase (GST), a junction sequence including a recognition site for a specific protease and a sequence able to encode an interferon alpha (IFN alpha) protein under the control of an inducible promoter, said vector encoding a GST-IFN alpha fusion protein
(2) Expressing said interferon alpha protein in conditions comprising the induction of the expression with 0.1 mM-0.5 mM IPTG and a growth temperature of 25° and/or 37°C, depending on said *E. coli* strain and
(3) Isolating the expressed IFN alpha protein.

## Description

The present invention relates to a method for the production of high-level soluble human recombinant interferon alpha (rhuIFNα) in *E. coli* and vectors useful for such a production.

Interferons (IFNs) are a group of naturally-produced glycoproteins endowed with antiviral, anti-proliferative, and immuno-modulatory properties (Pfeffer, 1997; Pestka *et al.,* 1987) as well as an analgesic action (Wang *et al.,* 2000; Wang *et al.,* 2002). The medical potential of IFNs was soon recognized, as demonstrated by the approval in 1986 of recombinant human IFNa2a (Roferon-A), and later IFNa2b (Intron-A), as drugs for the treatment of malignant and viral diseases (Gutterman *et al.,* 1994; Lauer *et al.,* 2001; Motzer *et al.,* 2002; Mahon *et al.,* 2002).

Most of the marketed pharmaceutical grade recombinant IFNα has since been produced and purified from *E. coli.*

The *E. coli* recombinant protein expression system has been, and still is, the system of choice for the production of IFNα. Indeed, IFNα genes do not have introns, and the protein products are generally not glycosylated. Furthermore, *E*. *coli* can grow rapidly to high cell densities, and strains used for recombinant protein production have been genetically modified so that they are generally regarded as safe for large-scale fermentation.

The expression of IFNα cDNA was achieved directly in *E. coli* soon after it was first cloned (Nagata et al., 1980; Pestka et al., 1983; Goeddel et al. 1980; Mizoguchi et al., 1985; reviewed by Pestka et al., 1987; reviewed by Barron and Narula 1990).

Several promoter systems were chosen to achieve high intracellular expression levels (Laplace *et al.,* 1988; Boyer *et al.,* 1992; Swaminathan *et al.,* 1999; Babu *et al.,* 2000; Lim *et al.,* 2000; Bedarrain *et al.,* 2001; Neves *et al.,* 2004, Srivasta et al. 2005).

However, IFNα protein expressed in large amount in *E. coli* often precipitate into insoluble aggregates called inclusion bodies (Swaminathan *et al.,* 1999; Bedarrain *et al.,* 2001; Srivasta et al. 2005) that are, in general, misfolded proteins and thus biologically inactive (Villaverde and Carrio, 2003).

In many cases, refolding from inclusion bodies (Middelberg, 2002) is considered undesirable, because of the poor recovery yield and the requirement for optimization of the refolding conditions for each target protein. Furthermore, resolu-bilization procedures may not fully restore the folding of the protein, and therefore its optimal function.

Solubility is a key issue for the production of recombinant protein in heterologous expression systems. Soluble recombinant proteins are often properly folded, functional, and easier to purify than aggregated proteins from inclusion bodies.

Hence, maximizing the production of recombinant proteins in a soluble form is an attractive alternative to the *in vitro* refolding procedures. Furthermore, it has been shown that fusion proteins have the advantage of providing a more favourable gene construct organization, permitting high levels of soluble protein to be expressed (Kapust and Waugh, 1999) by reducing the propensity to drive the protein folding process towards creating inclusion bodies (Lilie *et al.,* 1998).

Two main approaches are generally used, separately or in combination, to favour expression of soluble recombinant proteins.

* **The first one** calls for optimization of environmental factors such as growth temperature, media and concentration of gene expression inducers (Baneyx and Mujacic, 2004). A well known approach to limit the *in vivo* aggregation of recombinant proteins consists of cultivation at reduced growth temperature. Lowering the concentration of the inducer, in expression systems using an inducible promoter, also contributes to enhancing the solubility of recombinant protein produced in *E. coli* (Schein, 1989). This strategy has proven effective in improving the solubility of a number of difficult-to-express proteins (Vasina and Baneyx, 1997). The aggregation reaction is favoured at high temperature because of the strong temperature dependence of hydrophobic interactions (Kiefhaber *et al.,* 1991). It was reported that the direct consequence of decreasing temperature is the partial elimination of heat shock proteases that are induced during foreign protein overexpression (Chesshyre and Hipkiss, 1989). Moreover, expression and activity of a number of *E. coli* chaperones are increased at low temperature around 30°C (Mogk *et al.,* 2002; Ferrer *et al.,* 2003). Thus, the increased stability and potential correct folding at low temperature are partially explained by these factors. Furthermore, it was reported that low induction levels have been found to increase soluble protein expression (Baneyx and Mujacic, 2004; Weickert *et al.,* 1996).

The use of low temperature, for instance, has the combined advantages of slowing down transcription and translation rates and of reducing the strength of hydrophobic interactions that contribute to protein misfolding. However, the drawback of this approach (as in the case with low inducer concentrations) is a reduction in productivity and

* **The second one** involves genetic engineering of the target protein (Makrides, 1996; Sorensen and Mortensen, 2005 (J. Biotechnol); Sorensen and Mortensen, 2005 (Microb. Cell. Fact*):*
- Use of weaker promoters: the drawback of this approach is a reduction in productivity.
- In several instances, the use of an affinity tag has been proposed; the obtained fusion proteins may protect the target protein from proteolysis and/or enhance the solubility of the target protein. Common affinity tags are the polyhistidine tag (His-tag), which is compatible with immobilized metal affinity chromatography (IMAC), the thioredoxin tag and the glutathione-S-transferase (GST) tag for purification on glutathione based resins. As regards the property of enhancing the solubility of the target protein, other tags have been proposed: maltose-binding protein (MBP) or N-utilizing substance A (NusA). Other affinity tags have also been reported to improve protein yield, to prevent proteolysis, and to increase solubility *in vivo* (Makrides, 1996; Sorensen and Mortensen, 2005). Although many tag proteins are highly soluble, they are not all effective as solubility enhancers. Folding and disulphide bond formation in the target protein is enhanced by fusion to thioredoxin in strains lacking thioredoxin reductase (*trxB*).

The separation of the recombinant target protein from the affinity tag or solubility enhancers, is achieved by site-specific proteolysis; two serine proteases, namely factor Xa and thrombin are usually extensively employed. However selection of optimal reaction conditions and a specific protease depends on the recombinant target protein.
- coexpression of molecular chaperones implicated in *de novo* protein folding. However, chaperone coexpression may also reduce the overall yield of recombinant protein.

Several other expression systems were used to overcome the problem of inclusion bodies and improve protein solubility for the expression of human recombinant IFNα. These include *Bacillus subtilis* (Palva *et al.,* 1983), *Streptomyces lividans* (Pulido *et al.,* 1986), methylotrophic yeasts such as *Pichia pastoris* (Hitzeman *et al.,* 1981; Tuite *et al.,* 1982; Liu *et al.,* 2001), Murine Myeloma NSo cells (Rossmann *et al.,* 1996), and baculovirus-infected insect cells (Maeda *et al.,* 1985). Most of these systems have allowed the expression of soluble human recombinant IFNa; however none of them allowed the yields obtained in *E. coli* within inclusion bodies.

Thus, although the production of recombinant proteins in *E. coli* is well established, there are numerous factors which may present obstacles for successful production and purification of soluble recombinant proteins (Baneyx and Mujacic, 2004), the main obstacle being inclusion body formation and proteolytic degradation.

As regards interferon alpha 2, the highest amounts of recovery are reported in Srivasta P. et al., 2005, in which the refolding and purification yield were found to be ~ 3g/l with 58% recovery. The conditions were the following: recombinant E. coli D115a cells containing a plasmid expressing IFNα (pRSET-IFNa) which had the IFN-a2b gene under the T7 promoter was coexpressed with a plasmid which carried the gene for T7 RNA polymerase under the heat inducible λP_{L} promoter. This two plasmids expression system was optimized with respect to heat shock time, media and time of induction in a first step; the IBs (inclusion bodies) represented ~ 40% of the total cellular protein; in a second step the IBs were isolated and purified through ion exchange followed by step refolding to give a final product yield of ~ 3g/l. This procedure constitutes a laborious procedure.

All the methods described in the prior art either are not adapted to interferon alpha or lead to low yields of interferon alpha.

Even though both Srivasta et al., 2005 and Babu et al., 2000 propose methods of solubilization and purification of IFNα from inclusions bodies (IBs), with a good yield, these methods are time consuming and costly.

The purification of soluble recombinant proteins is more cost effective and less time consuming than refolding and purification from inclusion bodies.

Accordingly, there remains a need for a method that enables the production of interferon alpha from bacterial cells in a high yielding and cost-effective manner.

The Inventors have developed a strategy to drive the expression of human IFN α and more precisely IFN α2b in *E. coli* from aggregated protein in the inclusion bodies to soluble and properly folded cytoplasmic protein.

In the context of the invention, the Inventors have surprisingly found that production of soluble recombinant human IFN alpha can be achieved in *E.coli* by using a fusion protein GST-IFNα; this solution represents a more economical and efficient method for preparing IFN alpha than the ones proposed in the here above cited documents.

Therefore, a first aspect of the invention relates to a method for preparing a recombinant interferon alpha protein by expression in *E. coli,* said method being characterized in that it comprises the steps of:
(1) Transforming a *E. coli* strain selected in the group consisting of *E. coli* protease deficient host strains and *E. coli* reductase deficient host strains with a recombinant expression vector comprising the sequence encoding the glutathione-S-transferase, a junction sequence including a recognition site for a specific protease and a sequence able to encode an interferon alpha (IFN alpha or IFN α) protein under the control of an inducible promoter, said vector encoding therefore a GST-IFN α protein,
(2) Expressing said interferon alpha protein in conditions comprising the induction of the expression with 0.1 mM-0.5 mM isopropyl-β-D-thiogalactopyranoside (IPTG) and a growth temperature of 25° and/or 37°C, depending on said *E. coli* strain and
(3) Isolating the expressed IFN alpha protein.

According to a first advantageous mode of carrying out the method of the invention, the junction sequence of said vector consists of the sequence CTG GTT CCG Z 1 TCC Z2, wherein Z1 represents a thrombin recognition site CGT GGM and Z2 represents CCG GAA TTC TGT (SEQ ID NO:3) or TGT; therefore, when Z2 represents SEQ ID NO:3, the junction sequence is represented by SEQ ID NO: and when Z2 represents TGT, the junction sequence is represented by SEQ ID NO:2.

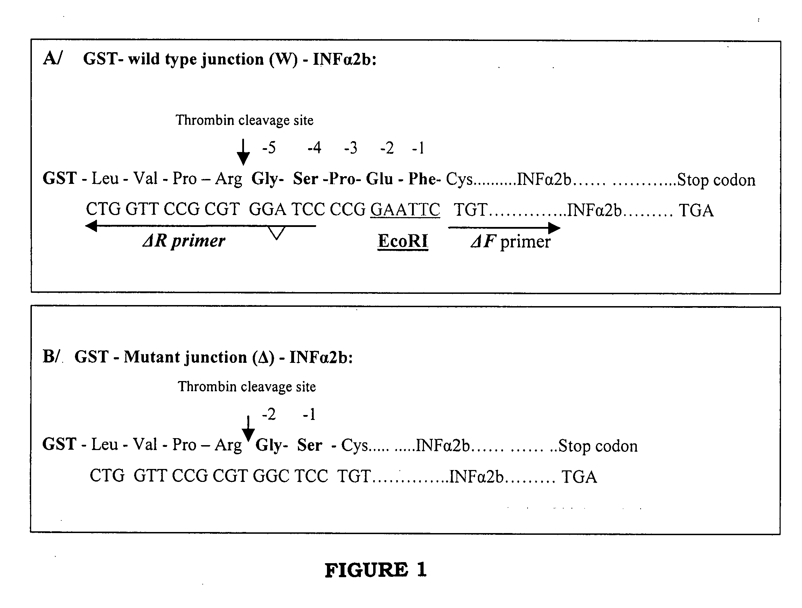

Two preferred following vectors may thus be obtained:
- vector WT which comprises the junction sequence CTG GTT CCG CGT GGA TCC CCG GAA TTC TGT (SEQ ID NO:4) (Z1= CGT GGM, with M=A and Z2= CCG GAA TTC TGT (SEQ ID NO:3)) and
- vector Δ which comprises the junction sequence CTG GTT CCG CGT GGC TCC TGT (SEQ ID NO:5) (Z1= CGT GGM with M=C and Z2 =TGT). This engineering consisted of deleting the three extra residues (Pro, Glu and Phe) at positions -1,-2 and -3 from the first amino-terminal residue of a human IFN alpha, preferably from IFN alpha 2b, and on optimizing the codon corresponding to the glycine at position -5 (figure 1). Furthermore said sequence includes a thrombin cleavage site (between CGT and GGC).

According to a second advantageous mode of carrying out the method of the invention, the *E. coli* protease deficient strain is an *E. coli lon⁻*/*ompT⁻* protease deficient host strain, preferably an *E. coli* BL21 strain.

According to a third advantageous mode of carrying out the method of the invention, the *E. coli* reductase deficient host strain is an *E. coli trxB⁻*/*gor⁻* reductase deficient host strains, preferably an *E. coli* Origami B strain DF5Δ/INF/IPT06, deposited at the CNCM (Collection Nationale de Culture de Microorganismes, 28 rue du Docteur Roux, 75015 PARIS) on April 30, 2007 under the accession number I-3760.

Surprisingly, the here above conditions lead to an enhancement (up to 70%) of the expression of IFNα as an intracellular soluble fusion protein in both *E. coli* strains.

However, in BL21 strain, this result was achieved only at a growth temperature of 25°C and induction with 0.1 mM-0.5 mM IPTG, whereas in Origami B cells, this result was achieved both at 25°C and at 37°C.

More precisely:
- when the thrombin recognition site is CGT GGA (i.e. M=A in Z1 and Z2 being either SEQ ID NO:3 or TGT as defined hereabove), the junction sequence: GTT CCG CGT GGA TCC Z2 may be either SEQ ID NO:6 or SEQ ID NO:7: when Z2 represents SEQ ID NO:3, the junction sequence is represented by SEQ ID NO:6 and when Z2 represents TGT, the junction sequence is represented by SEQ ID NO:7. This soluble GST-hIFNα2b fusion protein, even though obtained in great amounts, could not be digested efficiently with thrombin to release the IFN moiety. Inefficient thrombin digestion might be due to a sub-optimal folding of the fusion protein at the junction of GST and IFN. Furthermore, translational pausing was suspected as being the underlying cause for the presence of free GST. Possible reasons for translational pausing during protein expression in *E. coli* host strains were reviewed in the literature (Tsalkova *et al.,* 1999). It has been demonstrated that a strong correlation exists between the frequency of codon usage and the level of its cognate tRNA (Ikemura, 1981).
- when the thrombin recognition site is CGT GGC (i.e. M=C in Z1 and Z2 being either SEQ ID NO:3 or TGT as defined hereabove), the junction sequence: GTT CCG CGT GGC TCC Z2 (with Z2 preferably TGT) may be either SEQ ID NO:8 or SEQ ID NO:9: when Z2 represents SEQ ID NO:3, the junction sequence is represented by SEQ ID NO:8 and when Z2 represents TGT, the junction sequence is represented by SEQ ID NO:9). This soluble GST-hIFNα2b fusion protein leads to a significantly better cleavage, i.e., it was digested efficiently with thrombin to release the IFN moiety. After having analyzed the nucleotide and amino acid sequence at the junction between GST and IFN, the Inventors have designed the here above engineered sequence at this junction area to improve the thrombin cleavage, and eventually prevent free GST expression. Indeed, amino acid sequence analysis of the junction area revealed that the Pro at position -3 may influence the linker architecture, because its ring structure makes it more conformationally restricted and minimizes the flexibility of the thrombin recognition site. Moreover, the presence of a second Pro in the thrombin linker peptide sequence may form an X-Pro linkage, which is the most common cis peptide bond. This bond is known to show less preference for the trans configuration because the nitrogen of proline is bound to two tetrahedral carbon atoms, limiting the steric differences between the trans and cis form and resulting in a potential steric clash between the two adjacent GST and HuIFNα2b protein units. Moreover, because of the presence of the proline pyrrolidine ring, the Φ angle of rotation is blocked at approximately -65°, resulting in a restricted set of allowed Φ which mayangles, cause a freedom of rotation limit about the bond between the nitrogen and α-carbon. This may forbid the fusion protein to fold in many different ways. Furthermore, the steric clash occurring between the GST and huIFNα2b protein units may decrease the accessibility of thrombin protease towards its specific recognition site. Codon preference analysis at the junction area showed the presence of the rare glycine codon GGA (0.8%). Further more engineering was carried out to delete the Pro-Glu-Phe introduced by the EcoRI site to enhance the flexibility, and thus the accessibility of thrombin to the cleavage recognition site, and reduce the potentially immunogenic residues left after thrombin cleavage at the IFNa2b amino terminus. Changing the glycine codon GGA to GGC was carried out to prevent potential translational pausing.

Thus, according to a fourth advantageous mode of carrying out the invention, when the *E. coli* strain is an *E. coli* BL21 strain, the conditions of step (2) comprise a growth at 25°C and induction with 0.1-0.5 mM IPTG.

According to a fifth advantageous mode of carrying out the method of the invention, when the *E. coli* strain is an *E. coli* Origami B strain, the conditions of step (2) depend on the vector:
- when the vector includes the junction sequence SEQ ID NO:4 CTG GTT CCG Z1 TCC Z2, wherein Z1 represents a thrombin recognition site CGT GGA and Z2 represents CCG GAA TTC TGT (SEQ ID NO:3), the conditions of step (2) comprise a growth at 25°C and induction with 0.1-0.5 IPTG, preferably 0.5 mM IPTG;
- when the vector includes the junction sequence SEQ ID NO:5 CTG GTT CCG Z1 TCC Z2, wherein Z1 represents a thrombin recognition site CGT GGC and Z2 represents TGT, the conditions of step (2) comprise a growth at 25°C or 37°C and induction with 0.1-0.5 mM IPTG, preferably 0.5mM IPTG. The choice of *E. coli trxB⁻*/*gor⁻* Origami B as an alternative host was dictated by the fact that proper folding the IFN molecule necessitates the formation of two disulfide bridges between Cys 1-Cys 98 and Cys 29-Cys 138. Indeed, in the cytoplasm of normal *E. coli* strains, cysteines are actively kept in the reduced state by a pathway involving thioredoxin reductase and glutaredoxin (Ritz, *et al.,* 2001). Disruption of the *trxB* and *gor* genes, encoding the two major reductases of *E. coli,* allows formation of disulfide bonds in the *E. coli* cytoplasm (Xiong *et al.,* 2005). The *E. coli trxB⁻*/*gor⁻* Origami B strain has been selected in several expression situations where formation of disulfide bonds was successfully achieved (Bessette *et al.,* 1999; Lobel *et al.,* 2002; Lauber *et al.,* 2001; Venturi *et al.,* 2002). Surprisingly, close to 100% of GST-IFNα2b expressed by the Origami B from the engineered clone of the invention was soluble when culture was carried out at 37°C. This result shows that the *E. coli trxB⁻*/*gor⁻* Origami B strain is a good host for high expression level, soluble, and functional human IFNα2b from our engineered GST-IFNα2b clone. Cultivation of this clone at high cell density and at 37°C will probably allow the attainment of production level higher than the 300 mg/L of IFN that Babu *et al. 2000* obtained from inclusion bodies from optimized *E. coli* cultivation at high cell density.

High cell density fed-batch culture conditions of *E. coli* are for instance described in Yee L et al., 1992.

According to another advantageous mode of carrying out the method of the invention, said inducible promoter is the tac promoter. Other inducible promoters usable in *E. coli* may also be used, such as for instance the λP_{L} promoter.

According to yet another advantageous mode of carrying out the method of the invention, step (3) of isolating the expressed IFN alpha protein comprises successively, after lysis of the *E. coli* cells, centrifugation and retrieval of the supernatant:
- performing an affinity chromatography,
- performing a thrombin cleavage of the GST-IFN alpha fusion protein and
- performing a size exclusion purification for removing the glutathione and thrombin and obtaining said soluble and purified IFN alpha.

IFN alpha proteins are described for instance in Baron E. et al.; US Patent 5,710,027 and Nyman T.A. et al.; database accession numbers of the main IFN alpha proteins are the following: NM_024013; NM_000605; V00549; AY255838; NM_021068; NM_002169; NM_021002; NM_021057; NM_002170; NM-002171; NM_006900; NM_002172; NM_002173; NM_021268; NM_002175.

IFN alpha proteins which may be produced according to the instant invention may be any of the hereabove mentioned IFN alpha proteins or IFN alpha proteins having at least 70% of identity with one of the cited IFN alpha protein.

X% of identity between an IFN alpha protein P and a IFN alpha protein of reference R, means that when the two sequences are aligned, X% of the amino acids of P are identical to the corresponding amino acid in sequence R or are replaced by an amino acid of the same group:

### Amino acids with nonpolar R groups

Alanine, Valine, Leucine, Isoleucine, Proline, Phenylalanine, Tryptophan, Methionine.

### Amino acids with uncharged polar R groups

Glycine, Serine, Threonine, Cysteine, Tyrosine, Asparagine, Glutamine.

### Amino acids with charged polar R groups (negatively charged at Ph 6.0)

Aspartic acid, Glutamic acid.

### Basic amino acids (positively charged at pH 6.0)

Lysine, Arginine, Histidine (at pH 6.0).

Another grouping may be those amino acids with phenyl groups: Phenylalanine, Tryptophan, Tyrosine.

Another grouping may be according to molecular weight (i.e., size of R groups):

| | |
|---|---|
| Glycine | 75 |
| | |
| Alanine | 89 |
| Serine | 105 |
| Proline | 115 |
| Valine | 117 |
| Threonine | 119 |
| Cysteine | 121 |
| Leucine | 131 |
| Isoleucine | 131 |
| Asparagine | 132 |
| Aspartic acid | 133 |
| Glutamine | 146 |
| Lysine | 146 |
| Glutamic acid | 147 |
| Methionine | 149 |
| Histidine (at pH 6.0) | 155 |
| Phenylalanine | 165 |
| Arginine | 174 |
| Tyrosine | 181 |
| Tryptophan | 204 |

Particularly preferred conservative substitutions are:
- Lys for Arg and vice versa such that a positive charge may be maintained;
- Glu for Asp and vice versa such that a negative charge may be maintained;
- Ser for Thr such that a free -OH can be maintained; and
- GIn for Asn such that a free NH₂ can be maintained.

These percentages of sequence identity may be obtained using the BLAST program (blast2seq, default parameters) (Tatutsova and Madden, FEMS Microbiol Lett., 1999, 174, 247-250).

Surprisingly, the modifications of the expression plasmid (engineering of the GST-IFN junction including codon optimization) as well as the use of the modified *E. coli* expression strain *trxB⁻*/*gor⁻* double mutant, Origami B, allowed the production of 100 mg/L of pure, soluble, and functional recombinant hIFNα.

Therefore according to a sixth mode of carrying out the method of the invention, at least 100 mg/L of soluble IFN alpha is obtained. More than 300mg/L may be obtained in high cell density fed-batch cultures of *E. coli.*

According to another mode of carrying out the invention, the sequence encoding said interferon α protein is the sequence encoding interferon α2 protein. Said sequence encoding said interferon α2 protein comprises preferably the sequence encoding IFN α2b protein or a sequence encoding an interferon α2 protein which has more than about 70% identity with the IFN alpha2 protein of SEQ ID NO:15.

The invention also relates, in a second aspect, to a vector for expressing soluble interferon alpha in *E. coli,* characterized in that it comprises the sequence encoding the glutathione-S-transferase (GST), a junction sequence including a recognition site for a specific protease and a sequence able to encode an interferon alpha (IFN alpha) protein.

According to a first embodiment of carrying out said vector, the junction sequence of said vector consists of the sequence CTG GTT CCG Z1 TCC Z2, wherein Z1 represents a thrombin recognition site CGT GGM and Z2 represents CCG GAA TTC TGT (SEQ ID NO:3) or TGT (SEQ ID NO: 1 and SEQ ID NO:2).

According to one mode of carrying out said embodiment, the junction sequence consists of the sequence CTG GTT CCG CGT GGA TCC CCG GAA TTC TGT (SEQ ID NO:4) (Z1= CGT GGM, with M=A and Z2= CCG GAA TTC TGT (SEQ ID NO:3)).

According to another mode of carrying out said embodiment, the junction sequence consists of the sequence CTG GTT CCG CGT GGC TCC TGT (SEQ ID NO:5) (Z1= CGT GGM with M=C and Z2 =TGT).

According to a second embodiment of carrying out said vector, the sequence encoding said interferon α protein is a sequence encoding interferon α2. Said sequence encoding said interferon α2 protein comprises the sequence encoding IFN α2b protein.

The sequence of IFN alpha proteins are as defined hereabove.

Besides the above provisions, the invention also comprises other provisions which would emerge from the following description, which refers to examples of implementation of the invention and also to the attached drawings, in which:
- Figure 1: Design and Engineering of the GST-IFNα junction of the plasmid used to express soluble IFN in *E. coli.* Original wild type GST-IFNα junction, obtained following the cloning of IFNα2b in the pGEX4-T plasmid. The Pro-Glu-Phe residues were added to the IFNa2b N-terminus because of a cloning constraint. The vertical arrow shows the thrombin cleavage site, and the primers used for site directed mutagenesis of the junction are shown by the horizontal arrows ΔF and ΔR. The flipped triangle shows the *E. coli* rare GGA codon that was optimized. Engineered GST-IFN junction. The vertical arrow shows the thrombin cleavage site. Positions -1 and -2 are with respect to cysteine, the first residue of the IFNa2b.

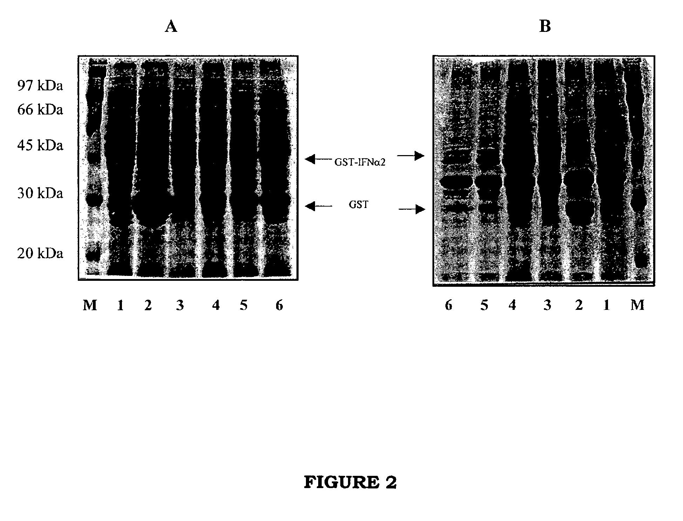

- Figure 2: Analysis of the recombinant GST-rhuIFNα2b expressed in the *E. coli* BL21 strain grown at 37°C. Both intracellular soluble **(A)** and insoluble **(B)** protein fractions were loaded on SDS/PAGE gels and proteins were stained with Coomassie brilliant blue R250. Lane M shows the molecular weight standards (RPN756 MW) indicated in KDa. Lanes 1 and 3 correspond, respectively, to the protein samples collected from an uninduced culture of *E. coli* BL21 transformed with pGEX4T-1 and *E. coli* BL21 transformed with the pGEX4T1/IFNα2b recombinant plasmid. Those lines serve as a control of protein expression in non-induced condition. Lane 2 corresponds to the protein sample collected from a 1 mM IPTG-induced culture of *E*. *coli* BL21 transformed with pGEX4T-1. This lane serves as a control of GST parental protein expression. Lanes 4, 5, and 6 correspond to the proteins collected from cultures of *E. coli* BL21 transformed with the pGEX4T1/IFNα2b recombinant plasmid induced respectively with 1, 0.5, and 0.1 mM IPTG.

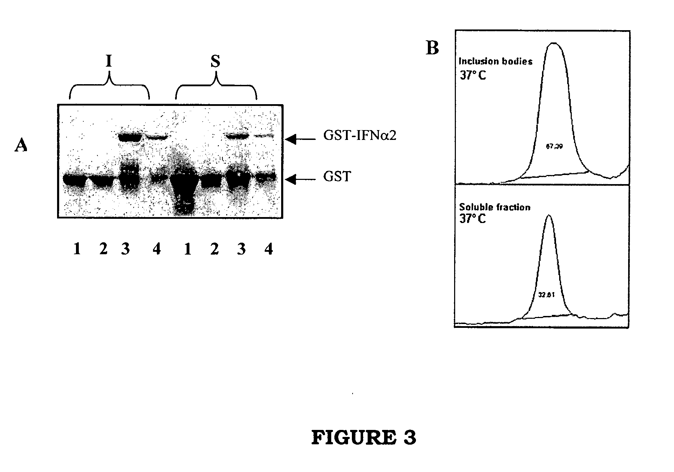

- Figure 3: Quantitative comparison between soluble and insoluble GST-IFNα expressed by *E. coli* BL21 grown at 37 °C. The BL21 host cell was grown to an OD_{600 nm} of 2 after induction with 0.1 mM IPTG at 37°C. A: Western blot analysis of both insoluble pellet (I) and soluble cytoplasmic fractions (S) using an anti-GST monoclonal antibody. Lanes (21, 2S) and (41, 4S) correspond, respectively, to the protein samples collected from uninduced cultures of *E. coli* BL21 transformed with the pGEX4T-1 plasmid and *E. coli* BL21 transformed with the pGEX4T1/IFNα2b recombinant plasmid. Those lines serve as a control of protein expression in uninduced conditions. Lane 1I and 1S correspond to protein samples collected from a 0.1 mM IPTG-induced culture of *E*. *coli* BL21 transformed with pGEX4T-1. This lane serves as a control of 26 kDa GST parental protein expression. Lane 3I and 3S correspond to protein samples collected from a 0.1 mM IPTG-induced culture of *E. coli* BL21 transformed with the pGEX4T1/IFNα2b recombinant plasmid. B: Comparison of soluble and insoluble GST-rhuIFNα2b fusion protein using the *ImageJ* software. Panel **B** shows that more than two-thirds of the GST-IFN fusion protein is present as insoluble aggregated protein when *E. coli* BL21 is cultured at 37°C and induced using 0.1 mM IPTG.

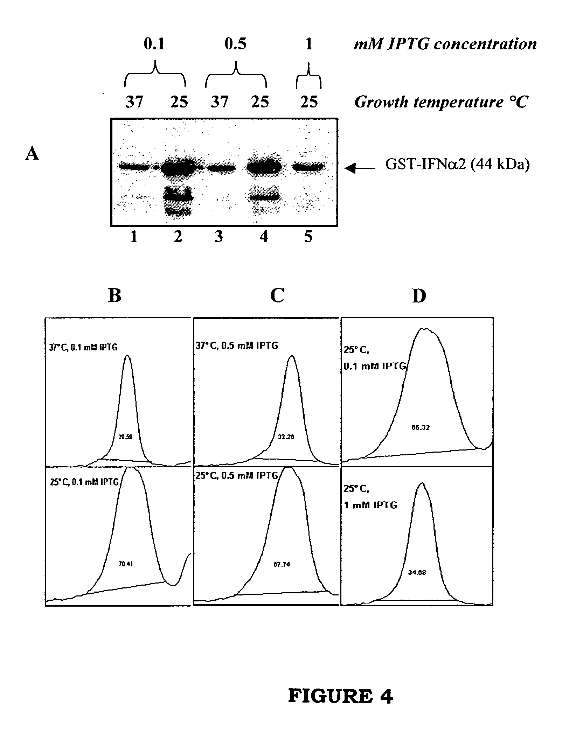

- Figure 4: Monitoring of the GST-rhuIFNα2b fusion protein expression in *E*. *coli* BL21 grown at 25°C and 37°C and induced using 0.1, 0.5 and 1 mM IPTG: (A) Western blot analysis using the Anti-human IFNα polyclonal antibody of soluble cellular proteins fractions from cultures carried out at: 37°C, 0.1 mM IPTG (lane 1), 25°C, 0.1 mM IPTG (lane 2), 37°C, 0.5 mM IPTG (lane 3), 25°C, 0.5 mM IPTG (lane 4) and 25°C, 1mM IPTG induction condition lane 5. 4B, C and D: Analysis by the *ImageJ* software of the soluble GST-rhuIFNα2b fusion protein expression in BL21 host strain grown at 25°C and 37°C.: Panel **B** shows the percentage of soluble GST-rHuIFNα2b fusion protein expressed by BL21 in cultures performed at 25°C and 37°C and induced with 0.1 mM IPTG. Panel **C** shows the percentage of soluble GST-rHuIFNα2b fusion protein expressed in cultures performed at 25°C and 37°C and induced with 0.5 mM IPTG. Panel **D** shows the effect of the IPTG concentration (0.1 mM and 1mM) on the expression in BL21 grown at 25°C, of soluble GST-rHuIFNa2b fusion protein.

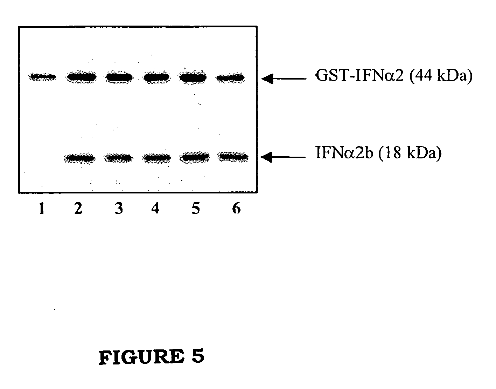

- Figure 5: Thrombin cleavage of soluble GST-wild type junction-IFNa2b fusion protein: Western blot using anti- human IFNα polyclonal antibody and showing the thrombin digestion products of 100 µg of GST-IFNα2b fusion protein carried out in a GSTrap affinity chromatographic column using 10U, 20U, 50U, 100U, and 200 thrombin units. Lane 1 shows the purified GST-IFNα2b fusion protein. Lanes 2, 3, 4, 5 and 6 show 10 µl of the thrombin cleavage products of GST-wild type junction-IFNa2b protein using increased amount of thrombin. The arrows represent the corresponding products after thrombin cleavage.

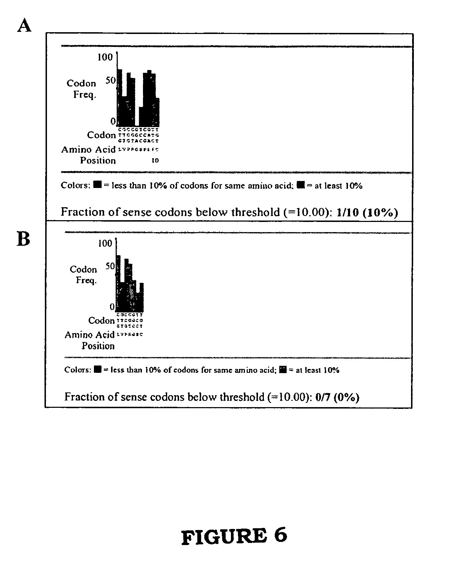

- Figure 6: Analysis of the codon usage at the junction GST-IFNα2b by the « *E. coli Codon Usage Analysis 2.0* » *software.* Panel **(A)** Analysis of the wild-type junction sequence shows the presence of the rare codon GGA (-5) encoding for the Glycine which has been classified as the eleventh rarest codon in *E. coli* mRNA, occurring at a frequency of 0.8%. Panel **(B)** shows the codon usage analysis of the modified junction (Δ) prediction analysis obtained PCR-Site directed mutagenesis.

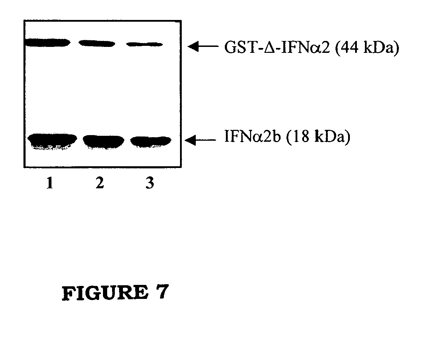

- Figure 7: Improved Thrombin cleavage at the engineered GST-IFNα2b junction. Western blot using Anti-human IFNα polyclonal antibody showing the results of the cleavage of GST-Δ-huIFNα2b with modified junction using 10U (**lane 1**), 20U (**lane 2**) and 50 unit (**lane 3**) thrombin protease. 10 µl of digested products were loaded on reducing SDS-PAGE followed by a Western blot-ECL Assay. The arrows represent the corresponding thrombin cleavage products.

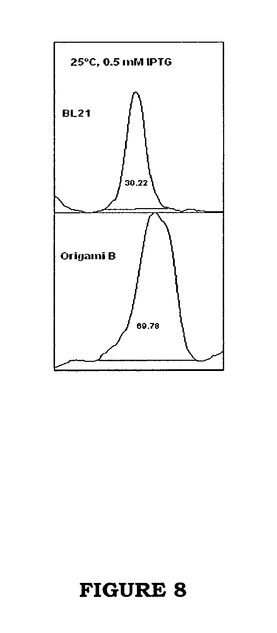

- Figure 8: Comparison using the *ImageJ* software of the level of soluble GST-IFNα2b expressed in both BL21 and Origami E. coli strain. *E. coli* Origami B cultures grown at 25°C and induced with 0.5mM IPTG expresses soluble GST-rHuIFNa2b twice as much as BL21 grown in the same condition.

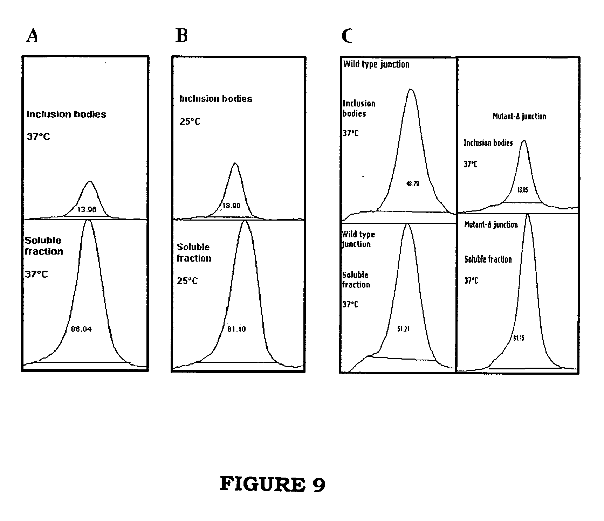

- Figure 9: Effect of the engineering of the GST-IFN and temperature of culture growth on the level of soluble GST-IFN α2b expressed by E. coli Origami B strain: **Panel A and B**, show that over 80% of the GST-rHuIFNα2b expressed by Origami B transformed by the plasmid with the modified GST-IFN junction and grown either at 37°C or 25°C is found in the soluble fraction. **Panel C**: shows that using the original GST-IFN junction and a growth temperature of 37°C, no more than half of the GST-rHuIFNa2b is found in the soluble fraction.

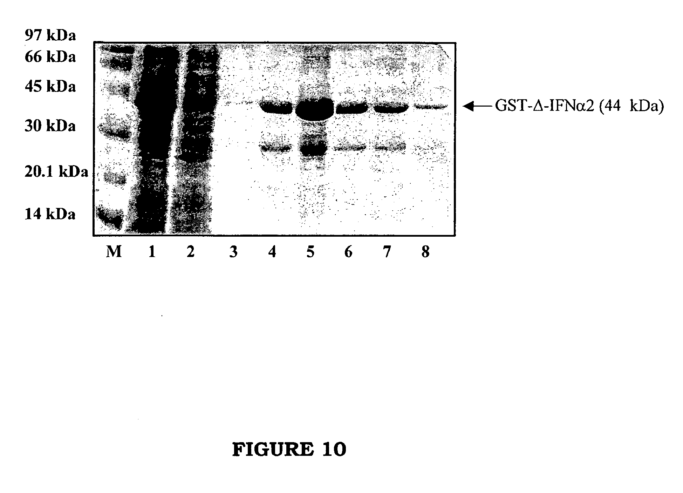

- Figure 10: Affinity chromatography purification on GSTrap column of soluble recombinant GST-Δ-huIFNα2b fusion protein: Coomassie stained SDS-PAGE showing lane 1, the soluble cellular fraction from an Origami B culture induced by 0.5 mM IPTG and grown at 25°C. Lane 2 corresponds to the flow-through fraction after the binding step on the affinity column. Lanes 3 to 8 correspond to the GST-Δ-rhuIFNα2b fusion protein eluted fraction with 50 mM Tris-HCI, 10 mM reduced glutathione, pH 8.0 elution buffer. Lane M shows the molecular weight standards RPN756 MW indicated in KDa.

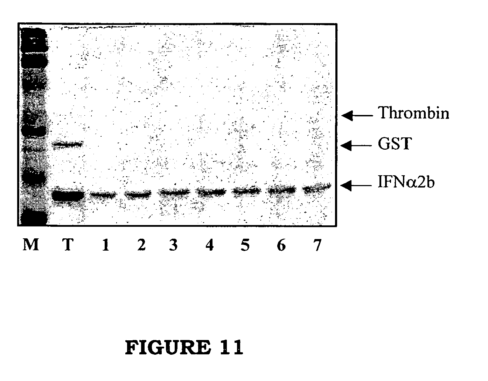

- Figure 11: Coomassie Blue stained SDS-PAGE analysis of purified rhuIFNa2b. Lane T shows the fraction after thrombin cleavage. Lanes 1 to 7 correspond to aliquots of the collected fractions during size exclusion chromatography on Sephacryl S-100 column. Lane M shows RPN756 Molecular weight marker. The arrows represent the corresponding products after thrombin cleavage.

The following examples illustrate the invention but in no way limit it.

### Example 1: Material and methods

### - Strains

The *E. coli* JM109/*recA⁻*, *endA⁻* strain (Stratagene) was used as the host strain for routine cloning experiments. The *E. coli trxB⁻*/*gor⁻* deficient strain, Origami B (Novagen, Madison, Wis), and the *E. coli lon⁻* and *ompT* BL21 protease-deficient strains (Amersham Pharmacia Biotech), were used as host strains for recombinant GST-Δ-huIFNα2b expression.

### - Construction of Recombinant pGEX-huIFNα2b expression vector

Human Interferon α2b cDNA was cloned by an RT-PCR approach using mRNA prepared from healthy individual leukocytes exposed *in vitro* to the Newcastle disease virus as described by *Wheelock et al. 1966* and *Waldmann et al. 1981,* using the TRIZOL^{™} mRNA extraction method as described by the manufacturer (Invitrogen). The cDNA corresponding to the IFNα2b published sequence (Pestka, 1983) was amplified using a forward primer that introduced an EcoRI site at the 5' end of the gene (5'-TGGAATTCTGTGATCTGCCTCAAACCCA-3' (SEQ ID NO:10)) and a reverse primer containing the XhoI site at the 3' end of the gene (5'-CGCTCGAGTCATTCCTTACTTCTTAAACTTTC-3' (SEQ ID NO:11)). The purified PCR product was digested with EcoRI and XhoI restriction enzymes and inserted into the plasmid pGEX4T1 (Amersham Biosciences) to generate the pGEX-huIFNa2b expression vector. Screening of pGEX4T1/IFNα2b recombinant plasmids containing the cDNA sequence encoding human IFNα2b was performed by a restriction mapping analysis using BgIII restriction enzyme as recommended by the manufacturer (Amersham Biosciences). Finally, the nucleotide sequence of the selected clones was checked by automated DNA Sequencing Analysis using the "ABI-PRISM ³⁷⁷" DNA sequencer (Perkin Elmer Applied Biosystems). The 5' pGEX sequencing primer (Amersham Biosciences) was used as the sequencing primer.

### - Construction of Recombinant pGEX-Δ-huIFNα2b expression vector

The pGEX-huIFNa2b expression vector was used as the DNA template for site-directed mutagenesis (PCR-SDM) procedures as described by *Rabhi et al. 2004,* using a pair of mutagenic primers (Genset-Oligos/Paris, France) as described in Fig. 1: ΔF (TGT GAT CTG CCT CAA ACC CAC (SEQ ID NO:12)) and ΔR (GGA GCC ACG CGG AAC CAG (SEQ ID NO:13)). Finally, screening of pGEX-Δ-huIFNα2b mutant clones was performed by restriction analysis using EcoRI restriction enzyme as recommended by the manufacturer (Amersham Biosciences), and by DNA sequencing analysis as described above.

### - Analytical expression of recombinant GST-rhIFNα2b

The Origami B and BL21 *E. coli* cell lines were transformed with the wild type pGEX-rhuIFNa2b plasmid and the GST-IFN junction reengineered pGEX-Δ-hIFNα2b plasmid, using the TSS method following standard protocols.

Starter cultures of 5 ml LB (Luria-Bertani) medium containing 100 µg/ml ampicillin were inoculated, each with a single *E. coli* Origami B or BL21 recombinant clone. The cultures were grown overnight at 250 rpm and 37°C. 1 ml of the overnight culture was added to 100 ml LB medium supplemented with 100 µg/ml ampicillin and further incubated at 37°C up to an OD₆₀₀ of 0.5.

### - Monitoring of growth conditions, i.e. temperature and IPTG concentration, using E. coli BL21 and Origami B strains

To check the effects of the inducer (IPTG) concentration and culture growth temperature on the expression of soluble GST-hIFNα2b wild type and GST-Δ-hIFNa2b mutant recombinant proteins, each host strain culture was induced with three IPTG concentrations (0.1, 0.5, and I mM) at an OD₆₀₀ of 0.5, and at temperatures of 25 °C and 37°C until reaching an OD at 600 nm of 2.

### - Extraction of GST-huIFNα2b recombinant protein

Cells from induced and uninduced cultures were harvested by centrifugation (4000g, 30 min, 4°C) followed by two washing steps with buffer A (10 mM Na₂HPO₄, 1.8 mM KH₂PO₄, 140 mM NaCl, 2.7 mM KCl, pH 7.3) at 4000g for 30 min, and finally stored at -70°C until use. Protein extraction was performed by resuspending the cell pellet in 1/5 of the original culture volume of buffer B (10 mM Na₂HPO₄, 1.8 mM KH₂PO₄, 140 mM NaCI, 2.7 mM KCI, pH 7.3, and 1 % Triton X-100). The cells were disturbed by six 30 second sonication steps. The supernatant was collected by centrifugation at 4°C for 30 min at 13500 rpm and stored for GST-Δ-huIFNa2b expression analysis. Finally, cell pellets corresponding to insoluble protein fractions (such as inclusion bodies) were washed separately with the same volume of buffer B.

### - Recombinant protein expression analysis

To analyze the intracellular expression of GST-Δ-huIFNα2b recombinant fusion protein in *E. coli* host cells, the clear supernatants were subjected to SDS-PAGE. Electrophoresis was performed using 15% SDS-polyacrylamide gels stained with Coomassie Brilliant Blue as described by Laemmli.

The recombinant fusion protein was detected by Western blot-ECL Assays (Amersham Biosciences) performed according to the manufacturer's instructions using either anti-GST peroxidase-conjugated sheep antibody at a dilution of 1:10,000 (Amersham Biosciences) or 1:400 dilution of Anti-human IFNα polyclonal antibody (ENDOGEN Searchlight), followed by the anti goat/sheep IgG peroxidase-conjugated monoclonal antibody (Sigma) used as the second antibody.

The ImageJ software was used to compare fusion protein expression under different growth conditions (e.g. IPTG concentration and growth temperature).

The concentration of GST-Δ-hIFNα2b in *Origami B* lysate versus rhuIFNα2b obtained after thrombin cleavage and the two-step purification was also determined by a quantitative in-house-developed ELISA assay. Dilution series containing 0 to 570 pg of HPLC-purified soluble IFNa2b produced in our laboratory were included in each assay to construct a standard curve. Recombinant proteins were detected using anti-human IFNα biotin-labelled monoclonal antibody (ENDOGEN Searchlight) and a colorimetric detection system using a streptavidin-horseradish peroxidase (HRP) conjugate (Amersham Biosciences).

The purity of the recombinant huIFNa2b was checked by analysis of 5µg recombinant protein on Coomassie-blue and silver-stained SDS-PAGE 15% gels.

### - Affinity chromatography step and Thrombin cleavage of GST-hIFNα2b recombinant protein

The supernatant containing the soluble GST-hIFNα2b recombinant protein was loaded on a GSTrap FF affinity column (1ml; Amersham Biosciences) pre-equilibrated with buffer A (10 mM Na₂HPO₄, 1.8 mM KH₂PO₄, 140 mM NaCI, 2.7 mM KCl, pH 7.3) at a flow rate of 1 ml/min at room temperature. The bound material was washed with buffer A until the absorbance at OD_{280 nm} returned to base line. Once the baseline was stable, elution of the bound GST-Δ-huIFNα2b recombinant protein was carried out using 6 column volumes of elution buffer (50 mM Tris-HCI, 10 mM reduced glutathione, pH 8.0) at a 0.5 ml/min flow rate. The eluted fractions containing the GST-huIFNa2b recombinant protein were pooled. The purification stages and affinity chromatographic profiles were analyzed by Coomassie blue-stained SDS-PAGE gels and by Western blot analysis as described above.

Twenty units of thrombin solution were added to 100 µg of eluted fusion protein and incubated at room temperature (+22 °C) for 20 hours.

### - Size exclusion purification step of hIFNα2b recombinant protein

Upon completion of thrombin digestion, the glutathione and thrombin were removed by a size exclusion chromatographic step. The digested product was loaded on a size exclusion Sephacryl S-100 26/60 High Resolution column (Amersham-Biosciences), and the cleaved rhuIFNα2b peak was eluted. The chromatographic profile was evaluated by Coomassie blue and silver-stained SDS-PAGE gels.

### - Biological activity of rhIFNα2b

The biological activity of the recombinant huIFNα2b preparation was determined by the antiviral and Gene Report assays as described by A. Meager, 2002, at the Division of Immunobiology, National Institute for Biological Standards and Control, UK. One unit of activity was defined as the amount of recombinant hIFNα2b required to produce antiviral activity equivalent to that expressed by 1 IU hIFNα2b reference standard (code: 95/566; Division of Immunobiology; National Institute for Biological Standards and Control, Potters Bar, UK).

Clone stability was checked after six months of continuous culture by plasmid DNA preparation and DNA sequencing of the expression cassette.

### Example 2: Cloning of the Human IFNα2b cDNA

Human Interferon α2b cDNA was cloned by an RT-PCR approach using mRNA prepared from the leukocytes of a healthy individual that were exposed *in vitro* to the Newcastle disease virus. The cDNA corresponding to the published sequence [38] was amplified using a forward primer that introduces an EcoRI site at the 5' end of the gene and a reverse primer containing the XhoI site at the 3' end of the gene. The purified PCR product was cloned between the EcoRI 5' end and XhoI 3' end of pGEX4T1, downstream of the sequence coding the Glutathione S-transferase (GST) gene (Fig.l), and under the control of the IPTG-inducible *tac* promoter. The nucleotide sequence of the selected clone was checked by automated DNA sequencing analysis using 5' pGEX sequencing primer.

### Example 3: Expression of GST-hIFNα2b fusion protein

The expression plasmid was introduced in *E coli lon⁻, ompT* BL21 and the *E. coli trxB⁻*/*gor⁻* deficient strain, Origami B. Monitoring of GST-huIFNα2b fusion protein expression was performed at 37 °C and at three IPTG concentrations (0.1, 0.5, 1 mM), and cell growth continued for 8 hours. The final OD at 600 nm was equal to 2 environ. The GST-hIFNα2b recombinant protein expression at 37 °C and at three different IPTG concentrations (0.1, 0.5, 1 mM) was analysed by 15% SDS-PAGE on both the supernatant and the cell pellet.

The expression profile of GST-hIFNα2b in *E. coli* BL21 strain is shown in Figure 2. The full fusion protein was present in both soluble (2A) and inclusion bodies fractions (2B). Furthermore, a band of 26 KDa corresponding to free GST was constantly observed. The expression of GST-huIFNa2b recombinant protein expression as inclusion bodies was highest at 37 °C and induction using a concentration of 1 mM IPTG. Therefore, an IPTG concentration of 0.1 mM was retained to compare the level of GST-hIFNα2b expression at 37 °C in both supernatant and inclusion body fractions by western blot analysis.

The presence of GST-huIFNa2b fusion protein in both the soluble and inclusion body fractions was confirmed by Western blot analysis using the anti-GST antibody (Fig. 3A). However the signal from the soluble fraction was much weaker as assessed by analysis of the western blot film using the ImageJ software. We observed an expression ratio of 67.39% insoluble (present in inclusion bodies) and over 32.61% soluble (present in the soluble fraction) GST-hIFNα2b fusion protein (Figure 3B).

Expression in *E. coli* Origami B strain of GST-hIFNα2b using the same plasmid gave a similar observation of higher ratio of insoluble recombinant protein at 37°C (data not shown).

### Example 4: Enhancement of the level of soluble GST-hIFNα2b expressed in BL21 strain at reduced temperature.

The effect of temperature and IPTG inducer concentration on the expression pattern of GST-hIFNα2b in *E. coli* strain BL21 was studied by western blot analysis. As shown in Figure 4A, the level of soluble GST-hIFNα2b expression increased when cell growth was carried out at 25°C and using 0.1 or 0.5 mM IPTG for induction, as compared to growth at 37°C. These observations were confirmed by analysis of the western blot films using the ImageJ software. The results shown in Figure 4B and 4C show that the amount of soluble intracellular GST-hIFN was increased more than twofold when culturing was performed at lower growth temperature (i.e. 25°C) and induction using either 0.1 mM or 0.5 mM IPTG. Furthermore, the level of soluble GST-hIFNα2b dropped to 34.68% when induction was carried out using 1 mM IPTG, even at 25 °C growth temperature (Fig. 4D).

### Example 5: Incomplete thrombin cleavage of GST-IFNα2b fusion protein

Five thrombin concentrations, 10U, 20U, 50U, 100U, and 200U, were used to cleave 100 µg of affinity-purified GST-IFNα2b fusion protein. The cleavage of the GST-hIFNα2b samples using the five thrombin concentration conditions was analysed on SDS-PAGE followed by a western blot analysis using anti-human IFNα polyclonal antibody. As shown in Figure 5, the cleavage rate was not complete and did not significantly increase when thrombin concentration was increased. Identical results of incomplete cleavage were observed using different sources of thrombin (i.e. different manufacturers).

### Example 6: Engineering of the GST-hIFNα2b expression cassette

To enhance the amount of the soluble GST-hIFNα2b protein expression and improve thrombin cleavage rate, we have engineered the sequence coding for the GST-IFN junction that includes the thrombin cutting site. As shown in Figure 1, this engineering consisted of deleting the three extra residues (Pro, Glu and Phe) at positions -1,-2 and-3 from the first amino-terminal residue of human IFN, and on optimizing the codon corresponding to the glycine at position -5.

The cDNA sequence corresponding to the thrombin recognition site was analyzed using the *« E.* coli Codon Usage Analysis 2.0 » software developed by Morris Maduro, which is available through the website http://www.lifesci.ucsb.edu/~maduro/codonusage/usage2.0c.htm. The codon sequence analysis (Fig. 6A and 6B) shows the presence of a rare glycine codon, GGA (-5), classified as the eleventh rarest codon in *E. coli* mRNA, occurring at a frequency of 0.8% (Wada *et al.* 1992). Site directed mutagenesis (Rabhi et al. 2004) was used to delete this codon from the GST-IFNα2b linker sequence, the Phe (at position -1) and Glu (at position -2) introduced in the original vector by the EcoRI cloning site, and the rigid Pro (at position -3) amino acid residues. Furthermore, the rare glycine codon, originally GGA, (-5) has been replaced by GGC according to the optimal codon usage for *E. coli.*

The engineered plasmid, called pGEX-Δ-hIFNα2b, was fully checked by DNA sequencing and used to transform *E. coli* BL21 and Origami strains.

### Example 7: Improvement of thrombin cleavage rate

To check the efficiency of thrombin cleavage of the engineered GST-IFN junction (sequence Δ), three thrombin concentrations (0U, 20U and 50U) were used to cleave 100 µg of affinity-purified GST-Δ-hIFNα2b fusion protein produced by BL21 grown at 25°C, and Origami B grown at 37°C. The cleavage samples from the three thrombin concentration conditions were analysed on SDS-PAGE gels followed by a western blot analysis using anti-human IFNα polyclonal antibody (Fig. 7). As shown in Figure 7, the cleavage of rhIFNa2b from the GST partner was optimal using 50 units of thrombin. This result shows that thrombin cleavage is improved when the junction of GST-Human IFNa2b is engineered.

### Example 8: Enhancement of the level of soluble rGST-huIFNα2b using the engineered clone

The original and engineered plasmids were introduced in BL21 (lon⁻ /ompT⁻) and Origami B (trxB⁻/gor⁻) *E. coli* strains, and the expression pattern of GST-hIFNα2b fusion protein was compared. Analysis was carried out by Western blot using the anti-GST antibody followed by ImageJ analysis. As shown in Figure 8, the amount of soluble GST-hIFNα2b protein was increased more than twofold when expression is carried out at 25°C and 0.5 mM IPTG using the Origami B host strain. Analysis of the expression of soluble GST-hIFNα2b fusion protein by the Origami B strain bearing the GST-IFN expression plasmid with the engineered junction, cultured at either 37°C (Fig. 9A) or 25°C (Fig. 9B), shows that over 80% of the protein was in the soluble fraction. It is interesting to note that the Origami B host strain is able to efficiently process the expression of soluble GST-hIFNα2b fusion protein at 37°C. Furthermore, when using the plasmid with the original GST-IFN junction sequence, the expression of soluble GST-hIFNα2b in Origami B could not reach more than 50% even when grown at the optimal conditions defined for Origami B (i.e. growth at 37°C and induction using 0.5 mM IPTG) (Fig. 9 C). This result shows that the expression of soluble form of recombinant GST-hIFNα2b depends on culture conditions (i.e. temperature, IPTG inducer concentration) but also on the host strain used for expression.

### Example 9: Purification of the soluble fraction of huIFNα2b expressed in E. coli

To measure the amount of GST-rhIFNα2b fusion protein produced by Origami B, the recombinant protein was purified from the supernatant of a culture grown at 37°C and 0.5 mM IPTG. Purification of GST-huIFNa2b was performed by affinity chromatography using a Glutathione Sepharose GSTrap column. The purification profile was analysed in a reduced, Coomassie stained SDS-PAGE gel (Fig. 10). A yield of **1.22 g/L** of purified GST-huIFNα2b was obtained.

The purified GST-hIFNα2b fusion protein was cleaved by thrombin protease to remove the GST moiety. Twenty units thrombin were used to digest 100 µg of GST-rhIFNα2b to completion (Fig. 11A, lane T and Fig. 11B, lane T). The 26 kDa GST moiety and the 37 kDa thrombin protease were removed by size exclusion chromatography. A final yield of **100 mg** of pure recombinant human IFNα2b was obtained. Purity was checked by Coomassie- stained SDS-PAGE gel (Fig. 11).

### Example 10: Biological activity of soluble huIFNα2b expressed in E. coli

The biological activity of the purified huIFNa2b preparation was determined by the antiviral and Gene Report assays (Meager, 2002). The antiviral assay is based on the ability of huIFNa2b to inhibit the cytopathic effect caused by encephalomyocarditis virus (EMCV) on the glioblastoma cell line 2D9. Furthermore, cell lines (HEK 293P) stably transfected with IFN-inducible promoter sequence (ISRE) linked to the SEAP gene (secreted alkaline phosphatase) were used to perform the Gene Report assay. The Relpot.xls Version 2.11 software [Scott Hutchinson/Amgen] was used to calculate the IFN biologic activity. The purified rhuIFNa2b from *E. coli* was calibrated against the IFNα WHO international standard (code: 95/566) (Meager, 2001) and exhibited a specific activity of 2x10⁸ IU/mg in both assays.

### References:

Babu, K.R., Swaminathan, S., Marten, S., Khanna, N. and Rinas, U. (2000) Appl. Microbiol. Biotechnol., 53, 655-660.
Baneyx, F. and Mujacic, M. (2004) Nat. Biotechnol., 22(11), 1399-1408.
Baron, E., and Narula, S. (1990). Critical reviews in Biotechnology., 10 (3), 179-190.
Bedarrain, A,. Cruz, Y., Cruz, O., Navarro, M. and Gil, M. (2001) Biotechnol. Appl. Biochem., 33, 173-182.
Bessette, P.H., Aslund, F., Beckwith, J. and Georgiou, G. (1999) Proc. Natl. Acad. Sci. USA, 96, 13703-13708.
Boyer, S.J., Colley, S.M., Lai, C.M., Swaminathan, N., Smith, W.A. and Beilharz, M.W. (1992) J Biol Regul Homeost Agents., 6 (3), 99-102.
Chesshyre, J.A. and Hipkiss, A.R. (1989) Appl. Microbiol. Biotechnol., 31, 158-162.
Ferrer, M., Chernikova, T.N., Yakimov, M.M., Golyshin, P.N. and Timmis, K.N. (2003) Nat. Biotechnol., 21,1266-1267.
Goedell, D.V., Yelverton, E., Ullrich, A., Heynker, H.L., Pestka, S. 1980. Nature., 287(5781), 411-416.
Gutterman, J.U. (1994) Proc. Natl. Acad. Sci. USA., 91(4), 1198-1205.
Hitzeman, R.A., Hagie, F.E., Levine, H.L., Goeddel, D.V., Ammerer, G. and Hall, B.D. (1981) Nature, 293(5835), 717-722.
Ikemura, T. (1981) J. Mol. Biol., 146(1), 1-21.
Kapust, R.B. and Waugh, D.S. (1999) Protein. Sci., 8, 1668-1674.
Kiefhaber, T., Rudolph, R., Kohler, H.H. and Buchner, J. (1991) Biotechnology (NY), 9, 825-829.
Laplace, F., Hartmann, M., Klessen, C., Tonew, M. and Malke, H. (1988) J. Basic Microbiol., 28(1-2), 55-61.
Lauber, T., Max, U.C., Schulz, A., Kreuzmann, P., Rosch, P. And Hoffmann, S. (2001) Prot. Express.Purif., 22, 108-112.
Lauer, G.M. and Walker, B.D. (2001) N. Engl. J Med., 345, 41-52.
Lilie, H., Schwarz, E. and Rudolph, R. (1998) Curr. Opin. Biotechnol., 9(5), 497-501.
Lim, H.K., Jung, K.H., Park, D.H. and Chung, S.I. (2000) App. Micrbiol. Biotech., 53, 201-208.
Liu, T.P., Tuan, V.T. and Villarete, L.H. (2001) Prot. Express. Purif., 22, 3 81-3 87.
Lobel, L., Pollak, S., Lustbader, B., Klein, J. and Lustbader, J.W. (2002) Prot.Express.Purif., 25,124-133.
Maeda S., Kawai, T., Obinata, M., Fujiwara, H., Horichi, T., Sacki, Y. and Furusawa, M. (1985) Nature, 315(6020), 592-594.
Mahon, F.X., Delbrel, X., Cony-Makhoul, P., Faberes, C., Boiron, J.M., Barthe, C., Bilhou-Nabera, C., pigneux, A., Marit, G. and Reiffers, J. (2002) J. Clin. Oncol., 20, 214-220.
Makrides, S.C. (1996) Microbiol. Rev., 60, 512-539.
Meager, A. (2002) J. Immunol. Methods., 261,21-36.
Meager A.,Gaines das R., Zoon. K. and Mire-Sluis A. Etablishment of new and replacement Word Health Organisation international Biological standards for human interferon alpha and omega (2001). J.immunol.Methods., 257(1-2):17-33.
Middelberg A. (2002) Trend. Biotechnol,. 20, 437.
Mizoguchi, J., Pitha, P., Raj, N.B.K. (1985). DNA., 4, 221-32.
Mogk, A., Mayer, M.P. and Deuerling, E. (2002) Chembiochem, 3,807-814.
Motzer, R.J., Bacik, J., Murphy, B.A., Russo, P. and Mazumdar, M. (2002) J. Clin. Oncol., 20, 289-296.
Nagata, S., Taira, H., Hall, A., Johnsrud, L., Streuli, M., Ecsodi, J., Boll, W., Cantell, K., Weissmann, C. (1980). Nature., 284, 316-320.
Neves, F.O., Ho, P.L., Raw, I., Pereira, C.A., Moreira, C. and Nascimento, A.L.T.O. (2004) Prot Express. Purif., 35, 353-359.
Nyman TA, Tolo H., Parkkinen J., Kalkkinen N. (1998) Biochem J., 329, 295-302.
Palva, I., Lehtovaara, P., Kaariainen, L., Sibakov, M., Cantell, K., Schei, C.H,. Kashiwagi, K. and Weissmann, C. (1983) Gene, 22, 229-235.
Pestka, S. (1983). Arch. Biochem.Biophys., 221 (1), 1-37.
Pestka, S. Arch. (1983) Biochem. Biophys, 221(1), 1-37.
Pestka, S., Langer, J.A., Zoon, K.C. and Samuel, C.E. (1987) Ann. Rev. Biochem., 56, 727-777.
Pfeffer, L.M. (1997) Semin. Oncol., 24(3), S9-63-S9-69.
Pulido, D., Vara, J.A. and Jimenez, A. (1986) Gene, 45(2), 167-174.
Rabhi, I., Guedel, N., Chouk, I., Zerria, K., Barbouche, M.R., Dellagi, K. and Fathallah, D.M. (2004) Mol. Biotechnol., 26(1), 27-34.
Ritz, D., Lim, J., Reynolds, M., Poole, L.B., Beckwith, J. (2001) Nature, 294, 158-160.
Rossmann, C., Sharp, N., Allen, G. and Gewert, D. (1996) Prot. Express. Purif. 7, 335-342.
Schein, C.H. (1989) Biotechnolgy, 7, 1141-1148.
Smith, D.B. and Johnson, K.S. (1988) Gene, 67(1), 31-40.
Sorensen, H.P. and Mortensen, K.K. (2005) J Biotechnol., 115, 113-128.
Sorensen, H.P. and Mortensen, K.K. (2005) Microb. Cell. Fact., 4(1): 1.
Srivasta, P., Bhattacharaya, P., Pandey, G., Mukherjee, K.J. (2005) Prot. Express. Purif 41, 313-322.
Swaminathan, S. and Khanna, N. (1999) Prot Express. Purif., 15, 236-242.
Tsalkova, T., Kramer, G. and Hardesty, B. (1999) J Mol. Biol., 286, 71-81.
Tuite, M.F., Dobson, M.J., Roberts, N.A., King, R.M., Burke, D.C., Kingsman, S.M. and Kingsman, A.J. (1982) EMBO. J, 1(5), 603-608.
Vasina, J.A. and Baneyx, F. (1997) Prot. Express. Purif, 9, 211-218.
Venturi, M., Seifert, C. and Hunte, C. (2002) J. Mol. Biol., 315, 1-8.
Villaverde, A. and Carrio, M.M. (2003) Biotechnol. Lett., 25, 1385-1395.
Wada, K.N., Wada, Y., Ishibashi, F., Gojobori, T. and Ikemura, T. (1992) Nucleic Acids Res. 20(1), 2111-2118.
Waldmann, A.A., Miller, R.S., Familletti, P.C., Rubinstein, S. and Pestka, S. (1981) Methods. Enzymol., 78(Pt A), 39-44.
Wang, Y.X., Jiang, C.L., Lu, C.L., Song, L.X., You, Z.D., Shao, X.Y., Cui, R.Y. and Liu, X.Y. (2000) J. Neuroimmunol., 108(1-2), 64-7.
Wang, Y.X., Song, L.H., Chen, Y.Z. and Jiang, C.L. (2002) Neuroimmunomodulation., 10 (1), 5-8.
Weickert, M.J., Doherty, D.H., Best, E.A., Olins, P.O. (1996) Curr. Opin. Biotechnol., 7,494-499.
Wheelock, E.F. (1966) J. Bacteriol., 92(5), 1415-1421.
Xiong, S., Wang, Y.F., Ren, X.R., Zhang, M.Y., Luo, Y., Zhang, L., Xie, Q.L. and Su, K.Y. (2005) World. J. Gastroenterol., 11(7), 1077-1082.

## Claims

1. A method for preparing a recombinant interferon alpha protein by expression in *E*. *coli,* said method being **characterized in that** it comprises the steps of:
(1) Transforming an *E. coli* selected in the group consisting of *E. coli* protease deficient host strains, and *E*. *coli* reductase deficient host strains, with a recombinant expression vector comprising the sequence encoding the glutathione-S-transferase (GST), a junction sequence including a recognition site for a specific protease and a sequence able to encode an interferon alpha (IFN alpha) protein under the control of an inducible promoter, said vector encoding a GST-IFN alpha fusion protein
(2) Expressing said interferon alpha protein in conditions comprising the induction of the expression with 0.1 mM-0.5 mM IPTG and a growth temperature of 25° and/or 37°C, depending on said *E*. *coli* strain and
(3) Isolating the expressed IFN alpha protein.

2. The method of claim 1, **characterized in that** the *E. coli* protease deficient strain is an *E. coli lon⁻*/*ompT* protease deficient host strain.

3. The method according to claim 2, **characterized in that** said strain is an *E*. *coli* BL21 strain.

4. The method of claim 1, **characterized in that** the *E. coli* reductase deficient host strain is an *E. coli trxB⁻*/*gor⁻* reductase deficient host strain.

5. The method of claim 4, **characterized in that** said strain is an *E. coli* Origami B strain.

6. The method according to claims 4 and 5, **characterized in that** the strain is deposited at CNCM (Collection Nationale de Culture de Microorganismes, 28 rue du Docteur Roux, 7501 PARIS) on April 30, 2007, under the accession number I-3760.

7. The method according to any one of claims 1 to 6, **characterized in that** the junction sequence of said vector consists of the sequence CTG GTT CCG Z1 TCC Z2, wherein Z1 represents a thrombin recognition site CGT GGM and Z2 represents CCG GAA TTC TGT (SEQ ID NO:3) or TGT (SEQ ID NO:1 and SEQ ID NO:2).

8. The method according to claim 7 **characterized in that** said vector comprises the junction sequence CTG GTT CCG CGT GGA TCC CCG GAA TTC TGT (SEQ ID NO:4) (Z1= CGT GGM, with M=A and Z2= CCG GAA TTC TGT (SEQ ID NO:3)).

9. The method according to claim 7, **characterized in that** said vector comprises the junction sequence CTG GTT CCG CGT GGC TCC TGT (SEQ ID NO:5) (Z1= CGT GGM with M=C and Z2 =TGT).

10. The method according to any one of claims 1 to 9, **characterized in that** when the *E. coli* strain is an *E. coli* BL21 strain, the conditions of step (2) comprise a growth at 25°C and induction with 0.1-0.5 mM IPTG.

11. The method according to any one of claims 1 to 9 **characterized in that** when the *E. coli* strain is an *E. coli* Origami B strain and when the vector includes the junction sequence SEQ ID NO:4 CTG GTT CCG Z1 TCC Z2, wherein Z1 represents a thrombin recognition site CGT GGA and Z2 represents the sequence CCG GAA TTC TGT (SEQ ID NO:3), the conditions of step (2) comprise a growth at 25°C and induction with 0.1-0.5 IPTG, preferably 0.5 mM IPTG.

12. The method according to any one of claims 1 to 9, **characterized in that** when the *E. coli* strain is an *E. coli* Origami B strain and when the vector includes the junction sequence SEQ ID NO:5 CTG GTT CCG Z1 TCC Z2, wherein Z1 represents a thrombin recognition site CGT GGC and Z2 represents TGT, the conditions of step (2) comprise a growth at 25°C or 37°C and induction with 0.1-0.5 mM IPTG, preferably 0.5 mM IPTG.

13. The method according to any one of claims 1 to 12, **characterized in that** said inducible promoter is selected from the group consisting of the tac promoter and the heat inducible λP_{L} promoter.

14. The method according to any one of claims 1 to 13 **characterized in that** step (3) of isolating the expressed IFN alpha protein comprises successively, after lysis of the *E. coli* cells, centrifugation and retrieval of the supernatant:
- performing an affinity chromatography;
- performing a thrombin cleavage of GST-IFN alpha protein and
- performing a size exclusion purification for removing the glutathione and thrombin and obtaining said soluble and purified IFN alpha protein.

15. The method of any one of claims 1 to 14, **characterized in that** at least 100 mg/L of soluble IFN alpha protein is obtained.

16. The method according to any one of claims 1 to 15, **characterized in that** the sequence encoding said interferon a protein is a sequence encoding interferon a2 protein.

17. The method of claim 16, **characterized in that** the sequence encoding said interferon a2 protein comprises the sequence encoding IFN α2b protein or a sequence encoding an interferon a2 protein which has more than about 70% identity with the IFN alpha2 protein of SEQ ID NO: 15.

18. A vector for expressing soluble interferon alpha in *E. coli,* **characterized in that** it comprises the sequence encoding the glutathione-S-transferase (GST), a junction sequence including a recognition site for a specific protease and a sequence able to encode an interferon alpha (IFN alpha) protein.

19. The vector according to claim 18, **characterized in that** the junction sequence of said vector consists of the sequence CTG GTT CCG Z1 TCC Z2, wherein Z 1 represents a thrombin recognition site CGT GGM and Z2 represents CCG GAA TTC TGT (SEQ ID N0:3) or TGT (SEQ ID NO:1 and SEQ ID NO:2).

20. The vector according to claim 18, **characterized in that** the junction sequence consists of the sequence SEQ ID NO:4 CTG GTT CCG CGT GGA TCC CCG GAA TTC TGT (Z1= CGT GGM, with M=A and Z2= CCG GAA TTC TGT (SEQ ID N0:3)).

21. The vector according to claim 18, **characterized in that** the junction sequence consists of the sequence SEQ ID NO:5 CTG GTT CCG CGT GGC TCC TGT (Z1= CGT GGM with M=C and Z2 =TGT).

22. The vector according to any one of claims 18 to 21, **characterized in that** the sequence encoding said interferon α protein is a sequence encoding interferon α2 protein.

23. The vector according to claim 22, **characterized in that** the sequence encoding said interferon α2 comprises the sequence encoding IFN α2b protein.


SEQUENCE LISTING

<110>  INSTITUT PASTEUR
       INSTITUT PASTEUR DE TUNIS

<120>  METHOD FOR THE PRODUCTION OF HIGH-LEVEL SOLUBLE HUMAN RECOMBINANT
        INTERFERON ALPHA IN E. COLI AND VECTORS USEFUL FOR SUCH A
       PRODUCTION

<130>  BLOcp226/141

<160>  15

<170>  PatentIn version 3.3

<210>  1
<211>  30
<212>  DNA
<213>  Homo sapiens


<220>
<221>  misc_feature
<222>  (15)..(15)
<223>  m = a or c

<400>  1
ctggttccgc gtggmtcccc ggaattctgt                                        30


<210>  2
<211>  21
<212>  DNA
<213>  Homo sapiens


<220>
<221>  misc_feature
<222>  (15)..(15)
<223>  m =  a or c

<400>  2
ctggttccgc gtggmtcctg t                                                 21


<210>  3
<211>  12
<212>  DNA
<213>  Homo sapiens

<400>  3
ccggaattct gt                                                           12


<210>  4
<211>  30
<212>  DNA
<213>  Homo sapiens

<400>  4
ctggttccgc gtggatcccc ggaattctgt                                        30


<210>  5
<211>  21
<212>  DNA
<213>  Homo sapiens

<400>  5
ctggttccgc gtggctcctg t                                                21

<210>  6
<211>  27
<212>  DNA
<213>  Homo sapiens

<400>  6
gttccgcgtg gatccccgga attctgt                                          27

<210>  7
<211>  18
<212>  DNA
<213>  Homo sapiens

<400>  7
gttccgcgtg gatcctgt                                                    18

<210>  8
<211>  27
<212>  DNA
<213>  Homo sapiens

<400>  8
gttccgcgtg gctccccgga attctgt                                          27

<210>  9
<211>  18
<212>  DNA
<213>  Homo sapiens

<400>  9
gttccgcgtg gctcctgt                                                    18

<210>  10
<211>  28
<212>  DNA
<213>  Artificial

<220>
<223>  forward primer

<400>  10
tggaattctg tgatctgcct caaaccca                                         28

<210>  11
<211>  32
<212>  DNA
<213>  Artificial

<220>
<223>  reverse primer

<400>  11
cgctcgagtc attccttact tcttaaactt tc                                    32

<210>  12
<211>  21
<212>  DNA
<213>  Artificial

<220>
<223>  mutagenic primer

<400>  12
tgtgatctgc ctcaaaccca c                                                21


<210>  13
<211>  18
<212>  DNA
<213>  Artificial

<220>
<223>  mutagenic primer

<400>  13
ggagccacgc ggaaccag                                                    18


<210>  14
<211>  504
<212>  DNA
<213>  Homo sapiens


<220>
<221>  CDS
<222>  (1)..(504)

<400>  14
atg tgt gac cta cca caa acc cac agc ctg ggt agc agg agg acc ttg        48
atg ctc ctg gcg cag atg agg aga atc tct ctt ttc tcc tgc ttg aag        96
gac aga cat gac ttt gga ttt ccc cag gag gag ttt ggc aac cag ttc        144
caa aag gct gaa acc atc cct gtc ctc cat gag atg atc cag cag atc        192
ttc aac ctc ttc agc aca aaa gac tca tct gct gct tgg gat gag acc        240
ctc cta gac aaa ttc tac act gaa ctc tac cag cag ctg aat gac ctg        288
gaa gcc tgt gtg ata cag ggg gtg ggg gtg aca gag act ccc ctg atg        336
aag gag gac tcc att ctg gct gtg agg aaa tac ttc caa aga atc act        384
ctc tat ctg aaa gag aag aaa tac agc cct tgt gcc tgg gag gtt gtc        432
aga gca gaa atc atg aga tct ttt tct ttg tca aca aac ttg caa gaa        480
agt tta aga tct aaa gag taa tga                                   504

<210>  15
<211>  166
<212>  PRT
<213>  Homo sapiens

<400>  15